# EUROPEAN PATENT APPLICATION

(11) **EP 3 766 423 A1**
(43) Date of publication of application: **20.01.2021**
(21) Application number: 19386034.3
(22) Date of filing: 19.07.2019
(51) Int. Cl.: A61B 5/16, A61B 5/00, A61B 5/11

(54) **DEVICE AND METHOD FOR DETECTING A POSSIBLE ABNORMAL HUMAN BEHAVIOUR**

(71) Applicant: Chatzatoglou, Vasilios, 166 72 Vari, Attica (GR)
(72) Inventor: Chatzatoglou, Vasilios, 166 72 Vari, Attica (GR)
(74) Representative: Yazitzoglou, Evagelia S.

(57) **Abstract**

A device (101) for detecting possible abnormal human behaviour is presented, comprising one or more kinetic sensors (10, 20, 30, 45) arranged in such a way that they cover a defined region of interest around the device (101), a CO₂ sensor (40), which measures the CO₂ concentration, a comfort feeling sensor (50) sensing the temperature and humidity and delivering a comfort feeling level around the device a sound loudness sensor (60), a processing module (70), a communication module (80), an action module (71) as well as a memory storage module (74), the processing module (70) being configured to process the data of the sensors by means of machine learning algorithms in order to build an individual dynamic nervousness model and to determine the actual nervousness grade in dependency of the model.

## Description

The invention is directed to a device for detecting a possible abnormal human behaviour according to the preamble of claim 1. Moreover, the invention is directed to a method for detecting a possible abnormal human behaviour, especially by means of the inventive device.

The present invention is based on the object of providing a device and a method for detecting possible abnormal human behaviour. The device should mainly work offline without the need to a permanent connection to the internet.

This object is achieved for a device by means of the features of claim 1. A method for detecting possible abnormal human behaviour by means of a device according to the invention is the subject matter of claim 6.

The device for detecting possible abnormal human behaviour comprises a plurality of sensors, a processing module, a communication module, an action module as well as a memory storage module.

According to an embodiment of the invention, the device comprises a one or more kinetic sensors arranged in such a way that they cover a defined region of interest e. g. 360° around the device with a desired elevation interval. The at least one kinetic sensor comprises one or more of pyroelectric sensors and/or microwave sensors.

The kinetic data provided by the at least one kinetic sensor are processed by the processing module so that vectored kinetic properties of a person in the defined region such as velocity, direction, frequency and time period are calculated. Microwave sensors are able to detect vectored human motion including a motion's direction, velocity, period and frequency.

The plurality of sensors also comprises one CO₂ sensor, which measures the CO₂ concentration as well as a comfort feeling sensor sensing the temperature and humidity and delivering a comfort feeling level around the device. Moreover, one sound loudness sensor is provided.

The signals of the sensors are transmitted to the processing module, whereby the grade of nervousness of a person as an indicator for possible abnormal human behaviour is calculated in dependency of the kinetic properties provided by the kinetic sensors, of the grade of changes of the CO₂ concentration provided by the breathing sensor, of the comfort feeling level based on the temperature and humidity values provided by the comfort feeling sensor and of the sound loudness provided by the sound loudness sensor.

The communication module is connected with the processing module and configured to communicate with a cloud and to transmit data to a server by using a standard for wireless communication such as GSM, Bluetooth® or WIFI for monitoring purposes.

The action module of the inventive device is connected with the processing module and able to create actions according to the evaluation of the nervousness grade and comprises a loudspeaker for playing sounds, RGB led indicators to indicate the nervousness grade and/or digital outputs for connection with an external alarm system.

The signals of the sensors are processed continuously in the processing module by means of machine learning algorithms in order to determine the nervousness grade as an indicator of possible abnormal human behaviour. The nervousness grade is stored in the memory storage module of the device as a function of time.

According to the invention the stored historical data are processed with statistical functions in order to create nervousness properties such as threshold values and weighting factors for calculating the nervousness grade based on the signals of the sensors within the framework of a learning process. During the learning process the machine learning algorithm generates a personal statistical dynamic nervousness model, which allows an evaluation of the actual nervousness grade and thus to detect the possible abnormal human behaviour in real time.

During operation of the device the current nervousness grade is determined in dependency of the historical data compared with the actual data.

At the start of the inventive method the sensors are calibrated, whereby after the calibration is performed real time data are transmitted from the sensors to the processing module. The data are being processed and stored in the memory module, whereby the data are used in order to build an individual nervousness model and to determine the actual nervousness grade in dependency of the model and the actual data provided.

The invention is explained in more detail by way of example in the following on the basis of the attached figures.

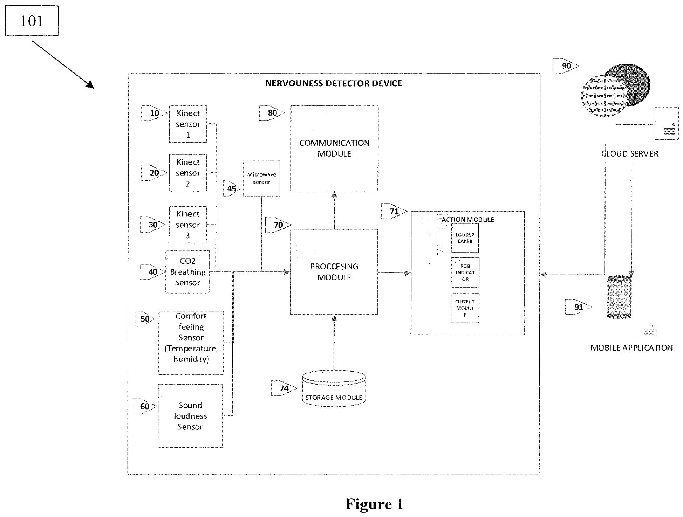

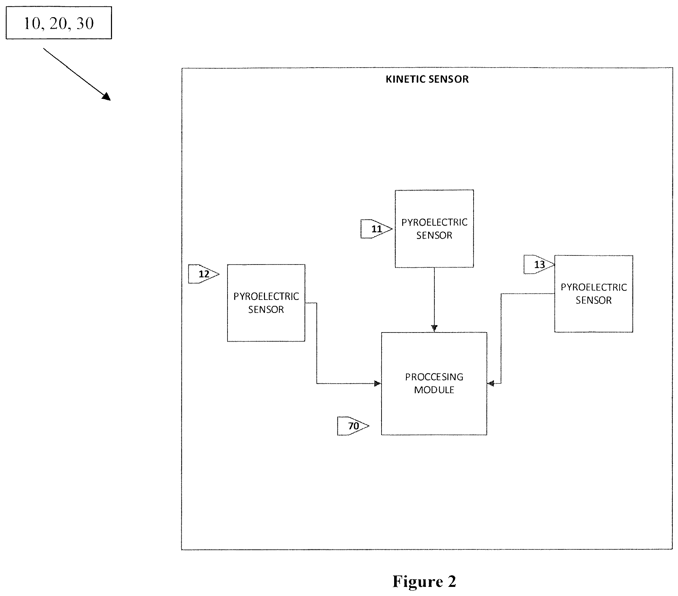

Figure 1 shows a schematic view of an embodiment of the inventive device, whereas figure 2 shows a flow chart of the inventive method.

According to the invention and referring to the embodiment shown in figure 1 a device 101 for detecting possible abnormal human behaviour comprises three kinetic sensors 10, 20, 20 arranged in such a way that they cover a defined region of interest around the device 101 with a desired elevation interval. In the embodiment shown in figure 1 and referring to figure 2 each of the kinetic sensors 10,20,30 comprises three pyroelectric sensors. The first kinetic sensor 10 comprises three pyroelectric sensors 11,12,13, the second kinetic sensor 20 comprises three pyroelectric sensors 21,22,23 and the third kinetic sensor 30 comprises three pyroelectric sensors 31,32,33.

In each of the kinetic sensors 10,20,30 the first pyroelectric sensor 11,21,31 has an 45° angle against the second pyroelectric sensor 12,22,32, which has an 45° angle against the third pyroelectric sensor 13, 23, 33, whereby the third pyroelectric sensor 13,23,33 has an 90° angle against the first pyroelectric sensor 11,21,31.

The second pyroelectric sensors 12,22, 32 in each kinetic sensor 10,20,30 are preferably used to confirm a valid completed human movement and filter faulty simple body motions.

The kinetic sensors can be kinect® sensors.

Within the framework of other embodiments the device 101 can comprise less than three kinetic sensors, each of them comprising three pyroelectric sensors as described above and shown in figure 2. For example the device can comprise one or two kinetic sensors. It is also possible than more than three kinetic sensors are provided. Within the framework of other embodiments each kinetic sensor comprises one or two pyroelectric sensors.

The device 101 optionally comprises a microwave sensor as shown in Figure 1. The microwave sensor 45 detects any human motion but not the human movement and it is used in order to confirm a valid human movement captured by the kinetic pyroelectric sensors 10,20,30.

The pyroelectric sensor assembly comprising the three kinetic sensors 10, 20, 20 transmits the collected data to the processing module 70 of the device 101, where the data are processed to detect period or velocity and direction of a completed human movement from one spot to another in a place, whereby noises coming of simple body motions and not from completed movements are filtered.

Within this context the data from the second pyroelectric sensors 12,22,32 of the kinetic sensors 10, 20, 20 are used to confirm a valid completed human movement and filter faulty simple body motions.

According to the invention the kinetic sensor data are processed in the processing module 70 in order to provide the period or velocity of human movement in one direction, the time period of a completed movement defined by two paired human movements with opposite direction and the frequency of actions, e.g. the frequency of two human movements with opposite directions in a specific time frame, e.g. 3 minutes. Also the time that elapses between two paired opposite movements, for example the time that someone takes to go to the kitchen and return, is calculated.

The period or velocity of human movement in one direction, the period of two paired human movements with opposite directions and the frequency of two paired human movements with opposite directions in a specific time frame, e.g. 3 minutes define the first three factors that affect the nervousness level in the algorithm according to the invention.

Referring to Figure 1 the device 101 also comprises a CO₂ sensor 40, which measures the rate of change of the CO₂ concentration in the room, where the device 101 is located and a comfort feeling sensor 50, which is sensing the temperature and humidity around the device 101. It should be noted that when the human being has high rate of movements, the CO₂ concentration is increased proportionally due to the breathing activity. As can be taken from figure 1 the device 101 also comprises one sound loudness sensor 60. The signal of the sound loudness sensor 60 and rate of change of the CO₂ concentration measured by the CO₂ reading sensor define the fourth and fifth factor that affect the nervousness level in the algorithm according to the invention. A sixth factor that affects the nervousness level in the algorithm according to the invention is the rate of change of the comfort feeling level captured by the comfort feeling sensor.

According to the invention the device comprises a storage module 74 and a communication module 80 adapted to communicate with a cloud and to transmit data to a cloud server 90 or to a mobile electronic device 91 by using a standard for wireless communication such as GSM, Bluetooth® or WIFI for monitoring purposes. In addition the device 101 comprises an action module 71 which creates actions according to the evaluation of the nervousness grade and comprises a loudspeaker for playing sounds, RGB led indicators to indicate the nervousness grade and/or digital outputs for connection with an external alarm system.

According to the invention by means of the plurality of sensors and of the processing module of the device 101 the six factors affecting the nervousness grade are determined and used in the machine learning algorithm. These six factors are being stored in the storage module during a continued learning process in order to enable the algorithm to generate a personal statistical dynamic nervousness model able to evaluate the nervousness level and to detect activities with an abnormal level.

The method for detecting a possible abnormal human behaviour contains four stages namely data acquisition, learning, evaluating and grading.

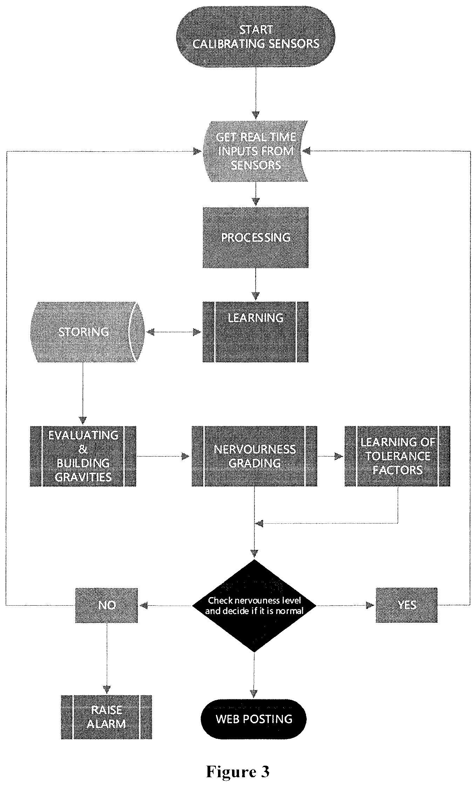

Referring to figure 3 at the beginning of the inventive method the sensors are calibrated, whereby after the calibration is performed in the data acquisition stage the sensors submit data to the processing module with a defined frequency, e.g. every 150 ms. Based on these data the processing module calculates the values for the six factors affecting the nervousness grade such as :
a) The period or velocity of human movement to one direction captured by one or more kinetic sensors;
b) The period of two paired human movements with opposite direction captured by one or more kinetic sensors;
c) The frequency of two paired human movements with opposite direction captured by one or more kinetic sensors during a specific time frame, e.g. 3 minutes;
d) The loudness level in db captured by sound loudness sensor;
e) The rate of change of the CO₂ concentration captured by the CO₂ sensor; and
f) The rate of change of the comfort feeling level captured by the comfort feeling sensor.

During the data acquisition stage, the system is storing in the storage module the raw values from the sensors in order to process them in the next stages.

In the learning stage, the system is being normalized by running a learning algorithm initially for a predefined long time period, e.g. for one week and then every predefined time interval for a shorter time period, e.g. every day in order to initialize the algorithm and then to update all the factors affecting the nervousness grade that are essential for the operation of the next two stages, namely the evaluating stage and the grading stage.

More specific the system is learning by processing the acquired data in order to create dynamic thresholds for the six factors affecting the nervousness grade and thus configuring the normal levels of the above six discrete factors affecting the nervousness grade.

The system is processing the long time period historical data using filters in order to exclude noises and then is using the average values to define a high threshold of normality for each factor affecting the nervousness grade. The thresholds as well as the historical data are being stored in the memory module.

Within the framework of the evaluation stage an evaluation algorithm is run once every predefined time interval, e.g. every day in order to evaluate the thresholds updated during the learning stage in order to build weightings for each of the six factors affecting the nervousness grade.

More specific the system in the evaluation stage is evaluating the high threshold of each factor affecting the nervousness grade by checking how many times during the predefined time interval a factor exceeds its high threshold, whereby the corresponding weightings are built as a result of the evaluation. The weightings range 0 to 1 in order to define how significant and precise the corresponding factors affecting the nervousness grade are. The most significant factor affecting the nervousness grade has the greater weighting.

The usage of the weightings is to affect and adjust the factors affecting the nervousness grade in the fourth stage of the method, namely in the grading stage.

In the grading stage, the system is running a grading algorithm every predefined time interval, e.g. every three minutes in which initially the nervousness level is calculated from the collected factors affecting the nervousness grade and their weightings in order to build a dynamic nervousness grade.

More specific the system is processing the collected data for each of the factors affecting the nervousness grade within a predefined time period, e.g. 3 minutes by checking if their values exceed their high thresholds and if this is the case then the algorithm raises an event by increasing a corresponding event counter for the factor affecting the nervousness grade.

After the predefined time period, the algorithm is processing the six event counters corresponding to the six factors affecting the nervousness grade by affecting them with the weightings for the factors affecting the nervousness grade, wherein the nervousness level is calculated based on the event counters. All the results are being stored in a statistical table.

According to the invention once every predefined time interval, e.g. every day the historical data from the statistical table are processed by running statistical functions like average, standard deviation, etc. in order to update a nervousness scale which is dynamic and adaptive according to the human behavior. The nervousness scale is following the changes in human behavior so when the human behavior is stable the nervousness scale will not change and the algorithm will build more precise the nervousness grading.

The dynamic nervousness scale depends on the day of the week and of the time of the day, so that according to another embodiment a learning procedure by using nervousness toleration factors in dependency of the day and the time is performed. Thus the system will tolerate high or low nervousness events as something to be expected and common for specific days and times. In case the nervousness grade exceeds an upper threshold or falls below a lower threshold an alarm by means of the communication module and/or the action module is raised.

For example in a case of a family with children, the algorithm learns that early in the morning from Monday to Friday and not for the other days of the week it is possible to detect high nervousness levels because of the preparations for the school and for the job. For these time periods high toleration factors are used but during the evenings the toleration is zero.

Another example is the case for senior parents, where for example the algorithm is being educated that every day they wake up early in the morning with normal nervousness and that they go to sleep early in the evening and also that they have a cleaning lady on Wednesday where high nervousness is to be expected and also that sometimes they have a visit from their children on Sunday with expected high nervousness.

So if a senior parent for example has a falling accident on Monday the algorithm will detect a nervousness level lower than the minimum and as the toleration factor is zero it will immediately raise a corresponding notification. Also, when a nervousness level greater than the maximum in a time zone with zero toleration is detected, a corresponding notification will be raised.

The results of the evaluation can be transmitted to a mobile electronic device or to a server in real time or upon request.

## Claims

1. Device (101) for detecting possible abnormal human behaviour, **characterized in that** it comprises one or more kinetic sensors (10, 20, 30, 45) arranged in such a way that they cover a defined region of interest around the device (101), a CO₂ sensor (40), which measures the CO₂ concentration, a comfort feeling sensor (50) sensing the temperature and humidity and delivering a comfort feeling level around the device a sound loudness sensor (60), a processing module (70), a communication module (80), an action module (71) as well as a memory storage module (74), the processing module (70) being configured to calculate
a) The period or velocity of human movement to one direction captured by one or more kinetic sensors (10, 20, 30, 45);
b) The period of two paired human movements with opposite direction captured by one or more kinetic sensors (10, 20, 30, 45);
c) The frequency of two paired human movements with opposite direction captured by one or more kinetic sensors (10, 20, 30, 45) during a specific time frame, e.g. 3 minutes;
d) The rate of change of the CO₂ concentration captured by the CO₂ sensor (40) and
e) The rate of change of the comfort feeling level captured by the comfort feeling sensor (50)
and to process the factors a)-e) together with the loudness level captured by the sound loudness sensor (60) by means of machine learning algorithms in order to build an individual dynamic nervousness model and to determine the actual nervousness grade in dependency of the model;
the communication module (80) being connected with the processing module and configured to communicate with a cloud and to transmit data to a server by using a standard for wireless communication for monitoring purposes; and
the action module (71) being connected with the processing module (70) and comprising a loudspeaker for playing sounds, RGB led indicators and/or digital outputs for connection with an external alarm system and being able to create actions according to the evaluation of the nervousness grade and to indicate the nervousness grade.

2. Device (101) for detecting possible abnormal human behaviour according to claim 1, **characterized in that** it comprises three or less kinetic sensors (10, 20, 20) arranged in such a way that they cover a defined region of interest around the device (101).

3. Device (101) for detecting possible abnormal human behaviour according to claim 2, **characterized in that** it comprises three kinetic sensors (10, 20, 20), each of the kinetic sensors (10,20,30) comprising three pyroelectric sensors, wherein in each of the kinetic sensors (10,20,30) the first pyroelectric sensor (11,21,31) has an 45° angle against the second pyroelectric sensor (12,22,32), which has an 45° angle against the third pyroelectric sensor (13, 23, 33), whereby the third pyroelectric sensor (13,23,33) has an 90° angle against the first pyroelectric sensor (11,21,31), the second pyroelectric sensor (12, 22, 32) in each kinetic sensor (10,20,30) being used to confirm a valid completed human movement and filter faulty simple body motions.

4. Device (101) for detecting possible abnormal human behaviour according to claim 2 or 3, **characterized in that** the kinetic sensors (10, 20, 30) are kinect® sensors.

5. Device (101) for detecting possible abnormal human behaviour according to claim 2, 3, or 4, **characterized in that** it comprises a microwave sensor (45) which detects any human motion and it is used in order to confirm a valid human movement captured by the kinetic sensors (10, 20, 30).

6. Method for detecting possible abnormal human behaviour by means of a device according to claims 1, 2, 3, 4 or 5 comprising the following steps after a calibration of the sensors (10, 20, 20, 40, 45, 50, 60) is performed:
in a data acquisition stage submission of the data of the sensors (10, 20, 20, 40, 45, 50, 60) to the processing module (70) with a defined frequency;
calculating and /or processing the values for the following six factors affecting the nervousness grade:
a) The period or velocity of human movement to one direction captured by one or more kinetic sensors;
b) The period of two paired human movements with opposite direction captured by one or more kinetic sensors;
c) The frequency of two paired human movements with opposite direction captured by one or more kinetic sensors during a specific time frame, e.g. 3 minutes;
d) The loudness level in dB captured by sound loudness sensor;
e) The rate of change of the CO₂ concentration captured by the CO₂ sensor; and
f) The rate of change of the comfort feeling level captured by the comfort feeling sensor,
whereby during the data acquisition stage, the raw values from the sensors are stored in the memory module (74) in order to be processed in the next stages;
in a learning stage running a learning algorithm initially for a predefined long time period, and then for a shorter time period every predefined time interval, in order to initialize the algorithm and to update all the factors affecting the nervousness grade, whereby the learning is performed by processing the acquired data in order to create dynamic thresholds for the six factors affecting the nervousness grade, wherein in the long time period historical data are processed using filters in order to exclude noises coming of simple body motions and not from completed movements and wherein the average values are used in order to define a high threshold of normality for each factor affecting the nervousness grade, the thresholds and the historical data being stored in the memory module (74)
in an evaluation stage running an evaluation algorithm once every predefined time interval in order to evaluate the thresholds updated during the learning stage in order to build weightings for each of the six factors affecting the nervousness grade, the high threshold of each factor affecting the nervousness grade being evaluated by checking how many times during the predefined time interval a factor exceeds its high threshold, whereby the corresponding weightings are built as a result of the evaluation;
and in a grading stage, running a grading algorithm every predefined time interval, in which initially the nervousness level is calculated from the collected factors affecting the nervousness grade and their weightings in order to build a dynamic nervousness grade, wherein the collected data for each of the factors affecting the nervousness grade are processed within a predefined time period by checking if their values exceed their high thresholds and if this is the case then the algorithm raises an event by increasing a corresponding event counter for the factor affecting the nervousness grade and wherein after the predefined time period, the event counters corresponding to the six factors affecting the nervousness grade are processed by affecting them with the weightings for factors affecting the nervousness grade and wherein the nervousness grade is calculated based on the event counters, all the results being stored in a statistical table.

7. Method for detecting possible abnormal human behaviour according to claim 6, wherein once every predefined time interval the historical data from the statistical table are processed by running statistical functions like average, standard deviation, etc. in order to update a nervousness scale which is dynamic and adaptive according to the human behavior.

8. Method for detecting possible abnormal human behaviour according to claim 7, wherein the dynamic nervousness scale depends on the day of the week and of the time of the day, so that a learning procedure by using nervousness toleration factors in dependency of the day and the time of the day is performed, enabling the algorithm to tolerate high or low nervousness grades as something to be expected and common for specific days and times, whereby in case the nervousness grades exceeds an upper threshold or falls below a lower threshold an alarm by means of the communication module (80) and/or the action module (71) is raised.
